# EUROPEAN PATENT APPLICATION

(11) **EP 2 574 905 A1**
(43) Date of publication of application: **03.04.2013**
(21) Application number: 11400045.8
(22) Date of filing: 29.09.2011
(51) Int. Cl.: G01N 15/06, G01N 15/02, G01N 33/28, G01N 21/31, G01N 21/53, G01N 21/71

(54) **Device and method for monitoring of particles in a lubricating circuit or a hydraulic system**

(71) Applicant: Eurocopter Deutschland GmbH, 86609 Donauwörth (DE)
(72) Inventor: Lees, Thilo, 86660 Tapfheim (DE); Vogel, Dominik, 86641 Rain am Lech (DE); Regenfelder, Florian, 80799 Munchen (DE); Schida, Silvio, 99310 Wipfratal (DE); Fuchs, Christian, 86641 Rain (DE); Gerstenhoefer, Manfred, 86655 Harburg (DE)
(74) Representative: Schmid, Rudolf

(57) **Abstract**

The invention is related to a device and a method for monitoring particles contaminating a lubricating circuit or a hydraulic system, particularly a lubricating circuit or a hydraulic system of an aircraft, such as a helicopter. Measuring means (12, 14, 15, 16) for sizes and quantities of particle (2) in a liquid medium of the lubricating circuit or of the hydraulic system comprise a luminous source (12) directing light into the liquid medium through optical windows (7, 8), at least one photo cell (15, 16) and at least one retaining means (9) for said particles (2).

## Description

The invention is related to a device for monitoring particles contaminating a lubricating circuit or a hydraulic system, particularly a lubricating circuit or a hydraulic system of an aircraft, such as a helicopter, with the features of the preamble of claim 1. The invention is further related to a method for monitoring particles contaminating a lubricating circuit or a hydraulic system, particularly a lubricating circuit or a hydraulic system of an aircraft, such as a helicopter, with the features of the preamble of claim 13.

A helicopter is provided with hydraulic systems to operate permanently the control of blade pitch, governing, etc.. Rotating components of a helicopter are driven by power transmission equipment, such as main gear box, front gear box, etc., with internal lubricating circuits to supply lubricant to the rotating components and to dissipate heat.

Known hydraulic systems supply pressure to the controls by means of a hydraulic medium. Each of the hydraulic systems is protected with filters against contamination of said hydraulic medium. If any of the filters are clogged, a pin indicates that a predetermined pressure difference across the filter has been exceeded by said hydraulic medium. This digital indication "on/off" does not give any information with regard to type and amount of the particles causing the choking and said indication by the pin is susceptible to errors, e. g. due to low temperatures. As the consequence of an indication by the pin is a lot of maintenance work, more detailed and safer information with regard to the contamination of the hydraulic systems would be welcome. Known lubricating circuits are equipped with sensors responsive to the oil pressure, oil temperature and responsive to swarf in the lubricant. Said sensors communicate their data to displays.

The document EP 2169381 A2 discloses a particulate matter detection device for an exhaust gas system. The device can measure the changes of the electric properties, thereby detecting the particulate matter adsorbed on the wall surface of a through hole.

The document EP 2095112 A1 discloses a device for monitoring particle contamination in flowing hydraulic systems comprising means for particle counting and particle sizing. Further a method for monitoring particle contamination in flowing hydraulic systems comprises the steps of determining (A, B) fluid flow velocity; counting of particles in the hydraulic system passing the light barrier for a fixed period of time; obtaining particle size distribution by using a range of different trigger levels. The monitoring device is insertable into the A/C hydraulic system to enable an online-monitoring of the degradation of the fluid quality during normal flight operations or on the ground.

The document US 4323843 A discloses an apparatus for detecting metallic contamination in a fluid such as engine transmission lubricant. The apparatus has two spaced apart electrodes, a magnetic flux extending between the electrodes the lines of force of which are substantially rectilinear, the electrodes being connectable to a circuit for signalling when an electrically conductive path is formed from one electrode to the other by metal particles attracted to the flux and formed into an electrode spanning bridge. The electrodes are connectable to circuit means whereby a change in interelectrode resistance may be detected.

The cited state of the art allows exclusively measurements with regard to quantities of contamination. The cited state of the art does not allow any assessment with regard to any origins of contamination, namely the cited state of the art does not allow any assessment whether particles have their origin from components indispensable for operation or if said particles have their origin from components without essential contribution for the present operability of the contaminated system.

It is therefore an object of the present invention to provide a device and a method for monitoring of particles contaminating a lubricating circuit or a hydraulic system, particularly a lubricating circuit or a hydraulic system of an aircraft allowing assessment with regard to specific qualities of said particles.

The solution is provided with a device for monitoring of particles contaminating a lubricating circuit or a hydraulic system, particularly a lubricating circuit or a hydraulic system of an aircraft with the features of claim 1 and with a method for monitoring of particles contaminating a lubricating circuit or a hydraulic system, particularly a lubricating circuit or a hydraulic system of an aircraft with the features of claim 13.

According to the invention a device for monitoring of particles, such as metal swarf, contaminating a lubricating circuit or a hydraulic system, particularly a lubricating circuit or a hydraulic system of an aircraft, is provided with measuring means for particle sizes and quantities in a liquid medium of the lubricating circuit or of the hydraulic system. Said measuring means comprise optical windows in a duct of the lubricating circuit or the hydraulic system and a luminous source directing light through said optical windows into the liquid medium of the lubricating circuit or of the hydraulic system. At least one photo cell is arranged outside said optical windows, allowing said photo cell to detect any particles in the liquid medium of the lubricating circuit or the hydraulic system, namely particles retained by at least one retaining means for said particles inside the lubricating circuit or in the hydraulic system next to or in front of said optical windows. The luminous source, at least one of the optical windows and the at least one photo cell are aligned relative to the at least one retaining means for said particles such that light directed from the luminous source through the optical window into the liquid medium is reflected by the retained particles to transmit to the photo cell a profile of the retained particles. The inventive device for monitoring allows visualization of particles contaminating a lubricating circuit or a hydraulic system through the optical windows and preferably by means of displays connected to the at least one photo cell. Subsequently the inventive device for monitoring allows assessment regarding quantity, size and type of particles retained by said retaining means in the liquid medium and illuminated by the luminous source through the optical windows, such assessment may include finding out whether the material of the particle reflecting the light is metallic or not-metallic. The assessment providing for recognition of material of retained particles allows further assessment with regard to any origins of said retained particles, namely whether said retained particles have their origin from components indispensable for operation of e. g. the aircraft, or if said particles have their origin from components without essential contribution for the present operability of the contaminated system. The inventive device for monitoring allows permanent detection and survey of the turbidity of the liquid medium in the lubricating circuit or the hydraulic system by means of the luminous source directing a strong light through the optical windows in the duct towards the at least one photo cell. The inventive device for monitoring allows integration into the lubricating circuit or in the hydraulic system and/or completion of a device for monitoring and application to gear boxes. The inventive device for monitoring allows reduction of maintenance work and increase of availability of the system operated by the inventive device for monitoring.

According to a preferred embodiment of the invention two photo cells are provided behind said optical windows, said photo cells being staggered with 90° and one of said photo cells being arranged opposite to the lubricating circuit or to the hydraulic system with regard to the luminous source to allow a 3-dimensional visualization of the retained particle and a visualization of the turbidity by detecting as well the diffusion of the light from the luminous source in the liquid medium.

According to a further preferred embodiment of the invention the at least one retaining means are at least one magnet. Said at least one magnet is connected to an electric circuit with detection means recognizing and communicating when a retained metal swarf closes the electric circuit. Preferably rinse means are provided to free the magnet from adhering metal particles, such rinse means comprising preferably supply means for high-power current to the magnet via the electric circuit with detection means to supply high-power current to the magnet to burn any detected adhering metal particles.

According to a further preferred embodiment of the invention at least one of said photo cells is a radiation spectrometer for optical metal analysis allowing assessment of the type of material of any particle retained by the magnet. The light from the sparks issued at burning up of the material of a particle retained by the magnet is seized by the radiation spectrometer for optical metal analysis of the type of metal by measuring the wavelength of the light emitted at burning up of the material of the particle. Once the type of material of the retained particle is detected the origin of the retained particle in the lubricating circuit or in the hydraulic system can be derived. If said analysis of the material of the retained burned particle gives as for example a result that the retained particle is made of brass and therefore from a component that is not essential for the safety of e. g. the aircraft any consequences from the fact that the particle has been detected may be delayed and consequently mission availability of an aircraft may be improved and maintenance efforts may be reduced. If said analysis of the material of the retained particle gives as a result that the retained particle is made of steel and therefore from an element that is essential for the safety of e. g. the aircraft, appropriate countermeasures may be started, e. g. by the crew of the aircraft.

According to a further preferred embodiment of the invention the at least one retaining means are at least one filter in the lubricating circuit or in the hydraulic system. The filter is arranged next to the optical windows, the luminous source and the at least one photo cell being aligned relative to the at least one filter for said particles such that light from the luminous source is deviated by the particles in the filter to transmit to the photo cell a profile of said retained particles to allow visualization of particles retained by the filter.

According to a further preferred embodiment of the invention the radiation spectrometer is connected to at least one monitor, preferably at least one monitor with at least one processor unit to make the data from analysis of retained particles available, e. g. to the crew of the aircraft.

According to a further preferred embodiment of the invention the two photo cells are each respectively connected to at least one processor unit to allow analysis of the turbidity detected from dimming and diffusion of the light from the luminous source in the liquid medium.

According to a further preferred embodiment of the invention said optical windows are respectively provided with at least one grid pattern to allow measurement of the size of a retained and visualized particle.

According to a further preferred embodiment of the invention at least one diameter reduction is provided in the duct of the lubricating circuit or in the hydraulic system, the retaining means are located inside the at least one diameter reduction and pressure sensors are provided respectively upstream and downstream of said at least one diameter reduction to prevent early any clogging by detecting any pressure differences, e. g. across the filter.

According to a further preferred embodiment of the invention said optical windows and said magnet are located at said at least one diameter reduction.

According to a further preferred embodiment of the invention any of the detection devices and/or processor units communicate to displays.

According to a preferred embodiment of the invention a method for monitoring of particles in a lubricating circuit or a hydraulic system with a device for monitoring comprises the following steps:
- sensing pressures in the liquid medium upstream and downstream of retaining means for particle inside a duct of the lubricating circuit or the hydraulic system,
- comparing said sensed pressures to detect values of any pressure losses in the liquid medium across the retaining means,
- directing light from a luminous source through an optical window next to the retaining means inside the duct of the lubricating circuit or the hydraulic system and through a further optical window opposed to the optical window relative to the duct,
- measuring the light from the luminous source with photo cells at the optical windows outside the duct of the lubricating circuit or the hydraulic system,
- communicating the data measured by the photo cells to at least one processor unit and to at least one monitor and
- classifying the liquid medium and any retained particle by means of at least one processor unit and/or by means of at least one monitor according to the data measured by the photo cells.

According to a further preferred embodiment of the invention the method comprises the following steps:
- providing a magnet as retaining means,
- applying an electric tension to separate poles of the magnet,
- detecting a metal particle retained by the magnet by measuring a loss of electric tension between the poles of the magnet,
- applying a high-power current to the magnet to burn up the seized metal particle,
- analyzing the light emitted by the seized metal particle by means of a radiation spectrometer for optical metal analysis as a photo cell.

A preferred embodiment of the invention is presented with reference to the following description and with reference to the attached drawings.

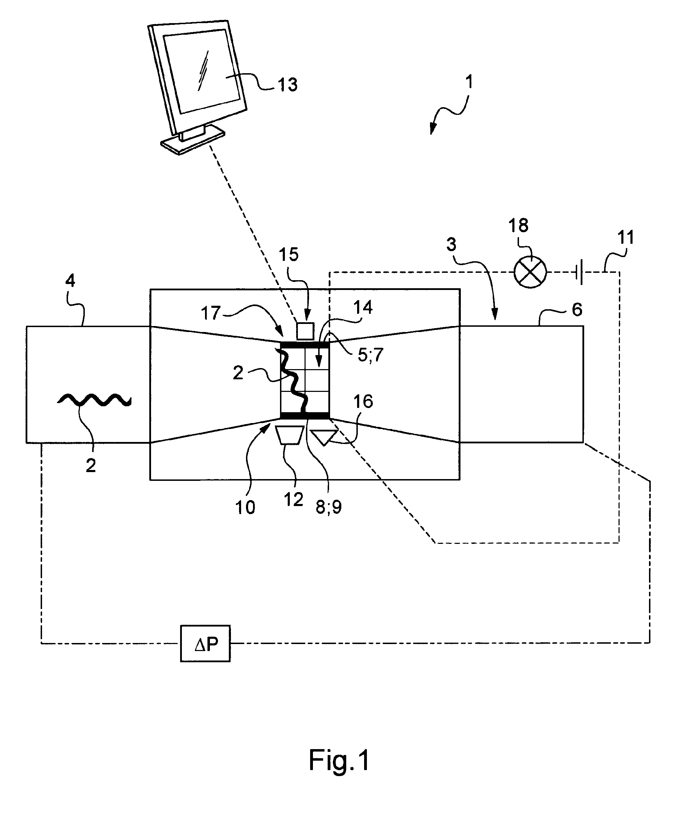

Fig. 1 shows a device for monitoring of particles according to the invention.

According to Fig. 1 a device 1 for monitoring of particles 2 is provided in the duct 3 of a lubricating circuit or a hydraulic system (not shown) of a helicopter. Oil as a liquid medium is flowing through the lubricating circuit or the hydraulic system from an inlet tube 4 through a coaxial tube 5 with a diameter reduction towards an outlet tube 6 of the device 1 for monitoring. Pressure sensors (not shown) are provided upstream and downstream of the coaxial tube 5 with diameter reduction to detect the respective pressure levels of the oil inside the duct 3 of the lubricating circuit or the hydraulic system. The pressure difference Δp of the pressures detected by the respective pressure sensors is surveyed to early forestall any chocking at the coaxial tube 5 with diameter reduction.

Opposed sections of the coaxial tube 5 with diameter reduction of the duct 3 are provided with optical windows 7, 8 as an element of measuring means. Further optical windows (not shown) provided in the coaxial tube 5 are staggered 90° to the opposed optical windows 7, 8. A grid 14 with a calibrated scale is provided for measurement of dimensions of any particles 2 visible from outside through the optical windows 7, 8 inside the lubricating circuit or the hydraulic system.

The coaxial tube 5 is equipped with a magnet 9 as retaining means. The magnet 9 has a negative pole 10 and a positive pole 17. The negative pole 10 and the positive pole 17 are respectively connected to an electric circuit 11 with detection means 18. The magnet 9 is adapted to retain metal particles 2 in the lubricating circuit or the hydraulic system. A metal particle 2 retained by the magnet 9 closes the electric circuit 11 and the detection means 18 signals said closure to a display, such as monitor 13. Subsequently a high-power current is supplied to the magnet 9 to burn the retained and detected metal particle 2 in order to rinse the magnet 9 and to emit a light with a wavelength characteristic for the material of the retained particle 2.

An alternative device for monitoring of particles 2 (not shown) comprises a coaxial tube that is equipped with a filter as retaining means instead of the magnet 9.

Radial outside of the coaxial tube 5 is arranged a luminous source 12. Opposite to the luminous source 12 with respect to the coaxial tube 5 is arranged a photo cell 15. A particle 2 retained by the magnet 9 or the filter is located in the array of the light directed from the luminous source 12 through the opposed optical windows 7, 8 to the photo cell 15.

A further photo cell 16 is conceived as a radiation spectrometer for optical metal analysis if the retaining means are the magnet 9. The radiation spectrometer 16 for optical metal analysis is arranged opposite to the photo cell 15 and next to the luminous source 12. The photo cells 15, 16 are respectively connected to processor units (not shown) and display 13 to visualize real time the retained particles 2 at the magnet 9 or in the filter. The processor units dispose of suitable software for said online assessment of size and/or type of material of the swarf. The data for said suitable software takes into account the materials provided in the lubricating circuit or the hydraulic system.

The radiation spectrometer 16 for optical metal analysis, used if the retaining means are the magnet 9, analyses the wavelength of the light emitted by any sparks when a retained and detected metal particle 2 is burned by the high-power current supplied to the magnet 9. An output of the radiation spectrometer 16 for optical metal analysis is connected to the processor units and display 13.

A temperature sensor (not shown) is provided in the lubricating circuit or in the hydraulic system. The temperature sensor is connected to the processor units and display 13.

### Method for monitoring of particles in a lubricating circuit or a hydraulic system

Oil flows through the duct 3 of a lubricating circuit or a hydraulic system lubricating, cooling and or driving a plurality of different components (not shown) of a helicopter such as rotors and/or gearboxes fabricated from different materials with specific coatings and supplements. Particles 2 detached from said different components are transported by the oil through the duct 3 to the device 1 for monitoring of particles 2.

The pressure sensors upstream and downstream of the coaxial tube 5 with diameter reduction detect the respective pressure levels of the oil inside the duct 3 of the lubricating circuit or the hydraulic system and produce a signal of the pressure difference Δp of the respective pressures detected upstream and downstream of the magnet 9 or the filter inside the coaxial tube 5. If the pressure difference Δp is too high, data is communicated to the display 13 to indicate a possible choking at the magnet 9 or the filter.

If a metal particle 2 is retained by the magnet 9 said metal particle 2 shortens the electric circuit 11 by connecting the negative and positive poles 10, 17. Such a closure of the electric circuit 11 is registered by the detection means 18 and communicated to the display 13. Said metal particle 2 is illuminated by the strong light from the luminous source 12 through the optical window 8 in spite of greatly contaminated oil in the coaxial tube 5 to create a picture of the retained particle 2 at the photo cell 15 outside the optical window 7. The data provided by the photo cell 15 is communicated to the display 13 via the processor unit with the size of the retained particle 2 derived by means of the grid 14 at the optical window 8.

A high-power current is supplied to the magnet 9 to burn the retained particle 2. While burning up the particle 2 emits light with a wavelength specific for the material(s) of the particle. The radiation spectrometer 16 for optical metal analysis derives real time data from said wavelength for online assessment whether a swarf as retained metal particle 2 has an origin in the lubricating circuit or the hydraulic system that is critical or uncritical for the safety of the helicopter and gives its output to the processor units and display 13.

The light directed from the luminous source 12 through the opposed optical windows 7, 8 and through the oil in the duct 3 to the photo cell 15 is used to assess the turbidity of said oil in the lubricating circuit or in the hydraulic system by comparing the brightness of the light of the luminous source 12 with the brightness of the light reaching the photo cell 15. The light from the luminous source 12 that is diffused by the oil in the lubricating circuit or in the hydraulic system is measured by the staggered photo cell for improved measurements of the turbidity of the oil in the lubricating circuit or in the hydraulic system. The output from the photo cell 15 and the staggered photo cell with regard to the turbidity of the oil is permanently transmitted real time to the processor units and display 13.

If a particle 2 is caught in the alternative device for monitoring of particles 2 by means of the filter said metal particle 2 is as well illuminated by the strong light from the luminous source 12 through the optical window 8 even if greatly contaminated oil is in the coaxial tube 5. A picture of the retained particle 2 is created at the photo cell 15 outside the optical window 7. The data provided by the photo cell 15 is communicated to the display 13 via the processor unit with the size of the retained particle 2 derived by means of the grid 14 at the optical window 8 and with data for the intensity of contamination at the filter. The turbidity of the oil in the lubricating circuit or in the hydraulic system is measured the same way as in the device 1 for monitoring of particles 2 by means of the magnet 9.

The temperature sensor provides temperatures to the processor units to even out data detected in the lubricating circuit or in the hydraulic system as a consequence of too low temperatures.

The optical analysis related to size and quantity of the retained particles and related to the type of the material, said particles are made of, is used to classify real time the contamination of the oil in the lubricating circuit or in the hydraulic system as nonhazardous or as an incentive to land the helicopter as soon as possible. The data from the photo cells 15, 16 are used to assess aging of the liquid medium and its pollution.

### Reference List

device 1
particles 2
duct 3
inlet tube 4
coaxial tube 5
outlet tube 6
optical windows 7, 8
retaining means 9 (magnet)
negative pole 10
electric circuit 11
luminous source 12
monitor 13
grid 14
photo cell 15
further photo cell 16 (radiation spectrometer for optical metal analysis)
positive pole 17
detection means 18

## Claims

1. Device (1) for monitoring of particles in a lubricating circuit or a hydraulic system, particularly a lubricating circuit or a hydraulic system of an aircraft, with measuring means (12, 14, 15, 16) for sizes and quantities of particle (2) in a liquid medium of the lubricating circuit or of the hydraulic system, said measuring means (12, 14, 15, 16) comprising a luminous source (12) directing light into the liquid medium of the lubricating circuit or of the hydraulic system, **characterized in that**
- optical windows (7, 8) are provided in a duct (3) of the lubricating circuit or of the hydraulic system,
- the luminous source (12) is directing light into the liquid medium of the lubricating circuit or of the hydraulic system through said optical windows (7, 8),
- at least one photo cell (15, 16) is provided outside of at least one of said optical windows (7, 8),
- at least one retaining means (9) for said particles (2) are provided in the lubricating circuit or in the hydraulic system next to or in front of said optical windows (7, 8), and
- at least one of the optical windows (7, 8), the luminous source (12) and the at least one photo cell (15, 16) are aligned with the at least one retaining means (9) for said particles (2).

2. Device (1) for monitoring according to claim 1,
**characterized in that** two photo cells (15, 16) are provided outside said optical windows (7, 8), said photo cells (15, 16) being staggered with 90°.

3. Device (1) for monitoring according to claim 1,
**characterized in that** the at least one retaining means (9) are at least one magnet said at least one magnet (9) being connected to an electric circuit (11) with detection means (18) and preferably rinse means to free the magnet (9) from adhering particles (2) such rinse means comprising preferably supply means for high-power current to the magnet (9).

4. Device (1) for monitoring according to claim 3,
**characterized in that**, at least one of said photo cells (15, 16) is a radiation spectrometer for optical metal analysis.

5. Device (1) for monitoring according to claim 4,
**characterized in that** the radiation spectrometer is connected to at least one monitor (13), preferably via at least one processor unit.

6. Device (1) for monitoring according to claim 1,
**characterized in that** the at least one retaining means (9) are at least one filter in the lubricating circuit or in the hydraulic system.

7. Device (1) for monitoring according to claim 2,
**characterized in that** the two photo cells (15, 16) are each respectively connected to at least one monitor (13), preferably via at least one processor unit.

8. Device (1) for monitoring according to claim 1,
**characterized in that** said optical windows (7, 8) are respectively provided with at least one grid pattern (14).

9. Device (1) for monitoring according to claim 1,
**characterized in that** at least one diameter reduction is provided in the lubricating circuit or in the hydraulic system and pressure sensors are provided respectively upstream and downstream of said at least one diameter reduction.

10. Device (1) for monitoring according to claim 9,
**characterized in that** said optical windows (7, 8) and said magnet (9) are located at said at least one diameter reduction.

11. Device (1) for monitoring according to claim 6,
**characterized in that** a temperature sensor is provided next to or in the filter.

12. Device (1) for monitoring according to any of the preceding claims,
**characterized in that** any of the measuring means and/or processor units communicate to monitors (13).

13. Method for monitoring of particles in a lubricating circuit or a hydraulic system with a device for monitoring according to claim 1, the method comprising the following steps:
- Sensing pressures in the liquid medium upstream and downstream of retaining means (9) for particle (2) inside a duct (3) of the lubricating circuit or the hydraulic system,
- Comparing said sensed pressures to detect values of any pressure losses in the liquid medium across the retaining means (9),
- Directing light from a luminous source (12) through an optical window (8) next to the retaining means (9) inside the duct (3) of the lubricating circuit or the hydraulic system and through a further optical window (7) opposed to the optical window (8) relative to the duct (3),
- Measuring the light from the luminous source (12) with photo cells (15, 16) at the optical windows (7, 8) outside the duct (3) of the lubricating circuit or the hydraulic system,
- Communicating the data measured by the photo cells (15, 16) to at least one processor unit and to at least one monitor (13) and
- Classifying the liquid medium and any retained particle (2) by means of at least one processor unit and/or by means of at least one monitor (13) according to the data measured by the photo cells (15, 16).

14. Method for monitoring of particles according to claim 13, the method comprising the following steps:
- Providing a magnet (9) as retaining means,
- Applying an electric tension to separate poles (10, 17) of the magnet (9),
- Detecting a metal particle (2) retained by the magnet (9) by measuring a loss of electric tension between the poles of the magnet (9),
- Applying a high-power current to the magnet (9) to burn up the seized metal particle (2),
- Analyzing the light emitted by the seized metal particle (2) by means of a radiation spectrometer for optical metal analysis as a photo cell (16).
